# EUROPEAN PATENT SPECIFICATION

(11) **EP 4 091 539 B1**
(45) Date of publication and mention of the grant of the patent: **25.06.2025**
(21) Application number: 21757564.6
(22) Date of filing: 14.01.2021
(51) Int. Cl.: A61B 5/02, A61B 5/0225, A61B 5/021, A61B 5/022, A61B 5/00

(54) **WEARABLE ELECTRONIC PRESSURE SENSING DEVICE**
TRAGBARE ELEKTRONISCHE DRUCKMESSVORRICHTUNG
DISPOSITIF ÉLECTRONIQUE DE CAPTAGE DE PRESSION PORTABLE

(30) Priority: 20.02.2020 CN 202010105006
(43) Date of publication of application: 23.11.2022
(73) Proprietor: Huawei Technologies Co., Ltd., Shenzhen, Guangdong 518129, (CN)
(72) Inventor: FU, Xiaoyu, Shenzhen, Guangdong 518129 (CN); HUANG, Zhenlong, Shenzhen, Guangdong 518129 (CN); ZHOU, Linfeng, Shenzhen, Guangdong 518129 (CN)
(74) Representative: Pfenning, Meinig & Partner mbB
(86) International application number: PCT/CN2021/071739
(87) International publication number: WO 2021/164478

(56) References cited:
- EP-B1- 3 026 523
- CN-A- 104 434 053
- CN-A- 104 434 053
- CN-A- 104 739 387
- CN-A- 109 730 642
- CN-U- 208 144 999
- JP-B1- 5 780 505
- US-A1- 2003 212 335
- US-A1- 2017 172 476
- US-A1- 2019 282 169

## Description

This application claims priority to Chinese Patent Application No. 202010105006.0, filed with the China National Intellectual Property Administration on February 20, 2020 and entitled "ELECTRONIC DEVICE".

### TECHNICAL FIELD

This application relates to the field of blood pressure measurement technologies, and in particular, to an electronic device.

### BACKGROUND

As people's living standard continuously improves, a quantity of hypertension patients in China increases. People need to pay more and more attention to measurement of human body indicators such as blood pressure.

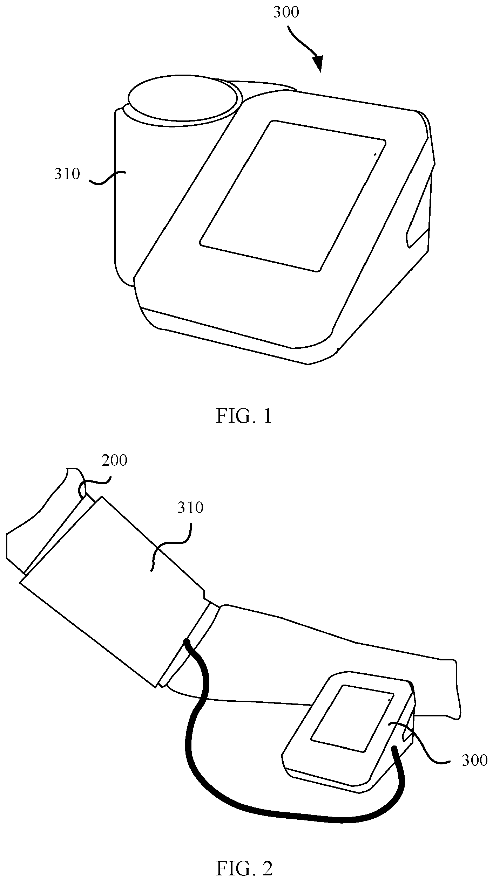

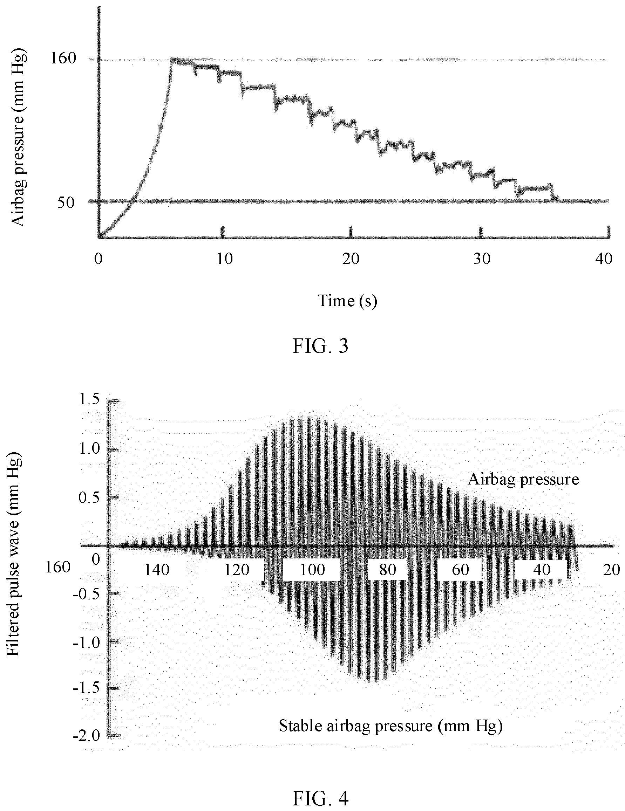

An oscillographic method is a blood pressure measurement method with high measurement precision that is widely used in current blood pressure measurement. As shown in FIG. 1 to FIG. 5, a detection principle of the oscillographic method is as follows: After a user wears a cuff 31, a micro pump is used to inflate an inner cavity of an airbag to pressurize and block a blood vessel 210 of a body part on which the user wears the cuff 31. Then, the micro pump releases air from the inner cavity to gradually stop blocking the blood vessel. In the foregoing process of inflating and releasing air, after a detected pressure wave signal in the inner cavity is filtered, an oscillation wave signal of air in the cuff 31 is obtained, and an oscillation wave envelope is obtained through fitting. In this case, a blood pressure value is estimated based on a relationship between the oscillation wave envelope and blood pressure. Currently, a common cuff blood pressure meter measures blood pressure by using a measurement principle of the oscillographic method. However, it is large and inconvenient to carry. Therefore, as shown in FIG. 4, in the conventional technology, an airbag 420 is disposed on a device body 410 of a wearable device 400, to form an existing wrist blood pressure meter. The wrist blood pressure meter is small, and is convenient for daily carrying and real-time detection. As the wrist blood pressure meter still uses the airbag 420 to press a body part of the user, the airbag 420 needs to be additionally disposed on the device body 410 of the wearable device 400.

However, the airbag 420 and the device body 410 of the wearable device 400 are two separate independent structures with poor integration. In addition, after the airbag 420 of the wearable device 400 expands to be an ellipsoid, an effective pressing length of a blood vessel is reduced. Consequently, precision of blood pressure detection is susceptible to disturbance of an interference signal, and is poor in stability. This affects accuracy of a detection result.

US 2003/212335 A1 discloses a pulse diagnostic instrument.

EP 3 026 523 B1 discloses an apparatus for contacting skin with sensor equipment.

CN 104 434 053 A1 discloses an electronic device for blood pressure measurement.

US 2019/282169 A1 discloses an electronic device that measures physiological information of a living subject.

### SUMMARY

This application provides an electronic device according to claim 1 that is wearable and further helps A device in accordance with the invention is defined in claim 1. improve accuracy of blood pressure measurement.

An embodiment of this application provides an electronic device, including a pressing component and a pressure sensor. The pressing component is disposed on a wearing structure of the electronic device. The pressing component includes an electromagnetic driving member and a pressure bearing member that may move under an electromagnetic force of the electromagnetic driving member. The pressure sensor is disposed on the pressure bearing member, and the pressure sensor is located on a surface that is of the wearing structure and that faces a measurement body part of a user. The pressure bearing member is configured to be driven by the electromagnetic driving member to press the pressure sensor on the measurement body part. The pressure sensor is configured to obtain a pulse wave signal of the measurement body part.

In this application, the pressing component and the pressure sensor are disposed on the wearing structure of the electronic device. The pressing component includes the electromagnetic driving member and the pressure bearing member, and the pressure bearing member may move under the electromagnetic force of the electromagnetic driving member. The pressure sensor is disposed on the pressure bearing member, and is located on a surface that is of the wearing structure and that faces the measurement body part of the user. In this way, according to one aspect, the pressure sensor may synchronously move with the pressure bearing member, and is driven by the electromagnetic driving member to press the measurement body part, to obtain a pulse wave signal of a measurement body part 0. This implements blood pressure measurement. In this application, the electromagnetic driving member may be precisely controlled, so that the pressure sensor presses the measurement body part accurately. This helps improve accuracy of blood pressure measurement. According to another aspect, both the pressing component and the pressure sensor are disposed on the wearing structure of the electronic device, so that the electronic device in this application has a higher degree of integration than an existing wearable device used for blood pressure measurement, and can facilitate daily carrying and real-time blood pressure monitoring. In this way, blood pressure management is implemented for the user. **In** addition, compared with an existing airbag pressing manner, in this application, the pressure sensor presses the measurement body part. This helps improve comfort of the measurement body part.

In a possible implementation, the pressure bearing member is located on a side that is of the pressure sensor and that is away from the measurement body part. In this way, when the pressure sensor is carried and fastened, the pressure sensor may be disposed close to the measurement body part, so that the pressure sensor presses the measurement body part. This further improves accuracy of blood pressure measurement.

In a possible implementation, when the pressure sensor is pressed on the measurement body part by the pressure bearing member, a movement direction of the pressure bearing member is from the electromagnetic driving member towards the measurement body part. In this way, according to one aspect, when blood pressure needs to be measured, the pressure sensor located on the pressure bearing member and the pressure bearing member move together towards the measurement body part, so that the measurement body part is pressed. According to another aspect, this improves aesthetics of the electronic device.

In a possible implementation, one of the electromagnetic driving member and the pressure bearing member includes an electromagnet, and the other includes a magnet. Magnetic poles of the electromagnet and magnetic poles of the magnet are disposed opposite to each other.

The electromagnet has an adjustable polarity, so that when poles of the electromagnet and poles of the magnet repel each other, the pressure sensor is pressed on the measurement body part, or when poles of the electromagnet and poles of the magnet attract each other, the pressure sensor leaves the measurement body part.

In this way, the polarity of the electromagnet may be changed to control movement of the pressure bearing member and the pressure sensor relative to the electromagnetic driving member. According to one aspect, the pressure sensor may press the measurement body part to obtain the pulse wave signal. When a blood pressure value is measured, a magnetic flux strength of the electromagnet may be precisely controlled, so that the pressure sensor presses the measurement body part accurately. This helps improve accuracy of blood pressure measurement. According to another aspect, the pressure sensor and the pressure bearing member may leave the measurement body part and return to the electronic device.

In a possible implementation, the pressure bearing member is fixedly connected to the pressure sensor or is detachably connected to the pressure sensor. In this way, the pressure sensor may be fastened to the pressure bearing member, and the pressure bearing member supports the pressure sensor. According to one aspect, when the pressure bearing member is driven by the electromagnetic driving member to move relative to the electromagnetic driving member, the pressure sensor may synchronously move with the pressure bearing member. According to another aspect, compared with an existing wearable device having a blood pressure measurement function, the electronic device may be enhanced in integrated structure.

In a possible implementation, the electromagnet includes a magnetic core and a coil wound around an outer peripheral side of the magnetic core. In this way, when a current is supplied into the coil, a magnetic field is generated around the coil, so that a polarity and a magnetic flux strength of the magnetic field are controlled to drive the pressure bearing member and the pressure sensor to move relative to the measurement par, and the pressure sensor presses the measurement body part accurately. This helps improve accuracy of blood pressure measurement.

The wearing structure includes a wrist strap and an electronic body. The wrist strap is connected to the electronic body, and the pressing component is disposed in the wrist strap.

In this way, when the pressure sensor performs blood pressure measurement by pressing the measurement body part, the pressing component, the pressure sensor, and the electronic device may be disposed in more diversified manners.

A mounting cavity is disposed on a surface that is in the wrist strap and that faces the measurement body part, and at least a portion of the pressing component is disposed in the mounting cavity. In this way, it is convenient to mount and fasten the pressing component and the pressure sensor, and the pressing component and the pressure sensor may be disposed close to the measurement body part. This facilitates pressing of the measurement body part and blood pressure measurement.

The pressure bearing member is slidably connected to an inner wall of the mounting cavity. In this way, when the pressure bearing member is mounted in the mounting cavity, it may be convenient for the pressure bearing member to be driven by the electromagnetic driving member to move. This reduces abrasion caused to the pressure bearing member and prolongs a service life of the electronic device.

The device further includes at least one sliding component. The sliding component is connected between the pressure bearing member and the inner wall of the mounting cavity, so that the pressure bearing member is slidably connected to the inner wall of the mounting cavity.

In this way, when the pressure bearing member is slidably connected to the inner wall of the mounting cavity of the wrist strap, abrasion caused to the pressure bearing member or the mounting cavity when the pressure bearing member moves in the mounting cavity may be reduced, so as to prolong a service life of the electronic device.

In a possible implementation, there are a plurality of sliding components, and the sliding components are disposed in different directions on a peripheral side of the pressure bearing member. In this way, according to one aspect, sliding between the pressure bearing member and the mounting cavity may be more stable, and stability performance of the pressing component in the wrist strap may be enhanced. This helps improve a pressing effect of the pressure sensor. According to another aspect, connection manners of the pressure bearing member and the mounting cavity may be more diversified.

In a possible implementation, each sliding component includes a first pulley disposed on a side wall of the pressure bearing member and a slide rail disposed on the inner wall of the mounting cavity. The pulley and the slide rail are disposed opposite to each other, and the first pulley may slide along the slide rail, so that the pressure bearing member is slidably connected to the inner wall of the mounting cavity.

In this way, the sliding between the pressure bearing member and the mounting cavity may be smoother, and the pressure bearing member may slide according to a set track of the slide rail.

In a possible implementation, a limiting part is disposed at an opening of the mounting cavity, and the limiting part is correspondingly disposed outside the first pulley in a pressing direction. In this way, according to one aspect, a movable stroke of the pressure bearing member may be limited through the limiting part. According to another aspect, the first pulley may be shielded, so as to enhance aesthetics and sealing performance of the electronic device.

In a possible implementation, a guide member is further disposed between the pressure bearing member and the electromagnetic driving member. A first guide groove corresponding to the guide member is further disposed in the electromagnetic driving member. A first end of the guide member is connected to the magnet, and a second end of the guide member passes through the first guide groove and may move in an extension direction of the first guide groove.

In this way, the guide member slides in the first guide groove, so that movement of the pressure bearing member may be better guided.

In a possible implementation, the sliding component includes a second pulley, a connection arm, and a second guide groove. The second guide groove and the mounting cavity are fastened relative to each other, and an extension direction of the second guide groove is staggered with a pressing direction of the electromagnetic driving member. A first end of the connection arm is hinged with the pressure bearing member, the second pulley is disposed at a second end of the connection arm, and the second pulley can slide in the guide groove, so that the connection arm drives the pressure bearing member to move in the pressing direction.

In this way, sliding between the pressure bearing member and the mounting cavity may be smoother through the sliding component, and the moveable stroke of the pressure bearing member may be limited through the sliding component.

In a possible implementation, the second guide groove is located in the mounting cavity; or
the second guide groove is formed into a cavity in communication with the mounting cavity.

In this way, the sliding component and the electronic device may be disposed in more diversified manners when the second pulley slides in the second guide groove.

In a possible implementation, the wrist strap is a metal wrist strap or a fluoro rubber wrist strap. In this way, deformation of the wrist strap relative to the pressing component or the pressure sensor may be avoided, so that the pressing component is more securely connected in the wrist strap. This helps improve stability performance of the electronic device.

In a possible implementation, the pressure sensor is even with a surface of the wearing structure; or the pressure sensor protrudes from a surface of the wrist strap.

In this way, when the pressure sensor presses the measurement body part, the pressure sensor may be disposed in a more diversified manner. This helps improve diversity of the electronic device.

In a possible implementation, the device further includes a processor. The processor is configured to measure a blood pressure value of the user based on the obtained pulse wave signal. In this way, blood pressure of the user may be measured through the processor and the pressure sensor, so that the user monitors the blood pressure in real time and manages the blood pressure.

In a possible implementation, the device further includes a display. The display is electrically connected to the processor, and is configured to display the blood pressure value. In this way, the measured blood pressure value may be intuitively displayed through the display, so that it is convenient for the user to obtain the blood pressure value.

In a possible implementation, the device further includes a wireless communication module. The wireless communication module is electrically connected to the processor. In this way, communication with the wearing structure of the electronic device or an external device may be implemented through the wireless communication module.

In a possible implementation, the device includes the wearing structure, the pressing component, and the pressure sensor. The pressing component is disposed on the wearing structure, and the pressure sensor is disposed on the pressing component.

In this way, according to one aspect, the pressure sensor may press the measurement body part, so that the electronic device is wearable and has a blood pressure measurement function. According to another aspect, the pressure sensor may be assembled on the wearing structure through the pressing component. This helps improve an integrated structure of the electronic device.

### BRIEF DESCRIPTION OF DRAWINGS

FIG. 1 is a schematic diagram of a structure of a cuff blood pressure meter in the conventional technology,
FIG. 2 is a schematic diagram of a structure of the cuff blood pressure meter in FIG. 1 when the cuff blood pressure meter is worn on an upper arm of a user;
FIG. 3 is a schematic diagram of airbag pressure change of a cuff blood pressure meter during measurement;
FIG. 4 is a schematic diagram of a wave of an airbag pressure pulse wave formed by a cuff blood pressure meter during measurement;
FIG. 5 is a schematic diagram of an envelope curve of a pressure pulse wave obtained when a cuff blood pressure meter performs measurement;
FIG. 6 is a schematic diagram of a structure of a wearable device for blood pressure measurement in the conventional technology;
FIG. 7 is a schematic diagram of a structure of an expanded airbag of the wearable device in FIG. 6;
FIG. 8 is a schematic diagram of pressing a measurement body part before and after expansion in the wearable device in FIG. 6;
FIG. 9 is a schematic diagram of a structure of an electronic device according to an embodiment of this application;
FIG. 10 is a schematic diagram of wearing an electronic device on a wrist according to an embodiment of this application;
FIG. 11 is a schematic diagram of a structure of an electronic device from another perspective according to an embodiment of this application;
FIG. 12 is a partial sectional view that shows a part A of the electronic device in FIG. 11;
FIG. 13 is a top view of the electronic device in FIG. 12;
FIG. 14 is a schematic diagram of a structure of the electronic device in FIG. 12 during blood pressure measurement;
FIG. 15 is a principle block diagram of blood pressure measurement performed by an electronic device according to an embodiment of this application;
FIG. 16 is a schematic diagram of another structure of the electronic device in FIG. 12 according to an embodiment of this application;
FIG. 17 is a top view of the electronic device in FIG. 16 according to an embodiment of this application; and
FIG. 18 is a schematic diagram of a structure of the electronic device in FIG. 16 according to an embodiment of this application during blood pressure measurement.

### Description of reference numerals:

100-Watch; 10-Wearing structure; 11-Wrist strap; 111-Mounting cavity; 1111-Inner wall; 1112-Opening; 1113-Limiting part; 12-Electronic main body;
20-Pressing component; 21-Electromagnetic driving member; 211-Magnetic core; 212-Coil; 213-First guide groove; 22-Pressure bearing member; 221-Magnet; 222-Lifting platform; 23-Guide member;
30-Pressure sensor; 40-Sliding component; 41-First pulley; 42-Second pulley; 43-Connection arm; 44-Second guide groove;
50-Processor; 60-Display; 70-Wireless communication module; 80-Storage module; 90-Signal conversion module; 91-Digital/analog conversion module; 92-Analog/digital conversion module;
200-Measurement body part; 210-Blood vessel;
300-Cuff blood pressure meter; 31-Cuff; 400-Wearable device; 410-Device body; 420-Airbag; and 500-Mobile phone.

### DESCRIPTION OF EMBODIMENTS

Terms used in embodiments of this application are only used to explain specific embodiments of this application, but are not intended to limit this application.

Blood pressure (blood pressure, BP): refers to lateral pressure exerted on a sidewall of a blood vessel per unit area when blood flows inside the blood vessel. In other words, human blood pressure refers to pressure that is generated by a pulsating blood flow in a blood vessel and that is perpendicular to a wall of the blood vessel in a lateral direction. A peak value of the pressure is systolic blood pressure, and may alternatively be referred to as high pressure. A valley value of the pressure is diastolic blood pressure, and may alternatively be referred to as low pressure. Blood pressure is main driving force of blood flow. The human blood pressure needs to be kept within a normal fluctuation range, in other words, both systolic blood pressure and diastolic blood pressure need to be reported within the normal fluctuation range. Excessively high or excessively low blood pressure adversely affects human health. Take a normal adult as an example. The systolic blood pressure of the adult should be greater than 90 mm Hg and less than 140 mm Hg, and the diastolic blood pressure should be greater than 60 mm Hg and less than 90 mm Hg. As blood vessels are different, blood pressure is respectively referred to as arterial blood pressure, venous blood pressure and capillary blood pressure. Generally, blood pressure during human body measurement is arterial blood pressure.

Currently, as people's living standard continuously improves, a quantity of hypertension patients in China increases. As people's public awareness of health is not enough, daily inspection is not timely. In addition, patients are lack of scientific daily blood pressure monitoring and guidance, and do not have a clear understanding of an objective of blood pressure management and impact of life habits. In addition, grassroots medical institutions do not provide comprehensive professional services, and are lack of management methods for patients outside hospitals, so that patients' therapy adherence is poor. According to related data, 23.2% of adults suffer from hypertension, and 71% of stroke and 54% of myocardial infarction deaths are related to the hypertension. Therefore, people need to improve awareness of a degree of attention paid to health, and strengthen measurement of a human body indicator such as blood pressure.

Take a cuff blood pressure meter as an example. Refer to FIG. 1 to FIG. 5. The following briefly describes a principle of measuring blood pressure by using an oscillographic method.

Blood pressure measurement by using the oscillographic method: After a user wears a cuff 31, a micro pump (not shown in the figure) is used to inflate an inner cavity of an airbag of the cuff 31 to pressurize and block a blood vessel of a body part on which the user wears the cuff 31. Then, the micro pump releases air from the inner cavity to gradually stop blocking the blood vessel. In the foregoing process of inflating and releasing air, after an air pressure sensor detects a pressure wave signal in the inner cavity, an oscillation wave signal of air in the cuff 31 is obtained after the pressure wave signal is filtered, and an oscillation wave envelope is obtained through fitting. In this case, a blood pressure value is estimated based on a relationship between the oscillation wave envelope and blood pressure.

However, a conventional blood pressure measurement device such as a cuff blood pressure meter 300 is large and inconvenient to carry, so that it is difficult to implement a requirement of people for real-time blood pressure monitoring.

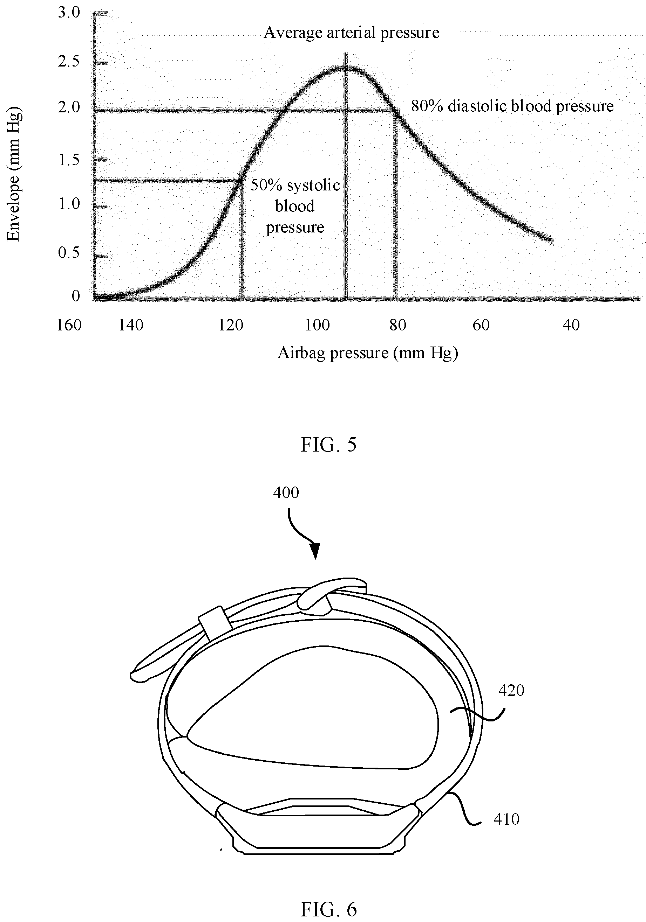

Therefore, in the conventional technology, an airbag 420 is disposed on a device body 410 of a wearable device 400, to form an existing wearable electronic device for blood pressure measurement, for example, a wrist sphygmomanometer, like a wrist sphygmomanometer shown in FIG. 6. The wrist sphygmomanometer is small and convenient for daily carrying and real-time detection. The wrist sphygmomanometer still uses a measurement principle of the oscillographic method, and uses the airbag 420 to press a body part of the user. For the wearable electronic device used for blood pressure measurement, the airbag 420 needs to be additionally disposed on the foregoing device body 410.

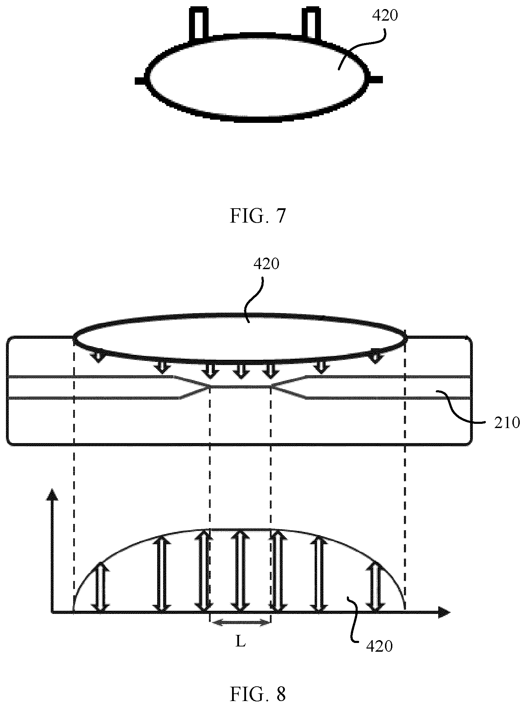

Refer to FIG. 6. The airbag 420, however, is usually disposed on a side that is of the device body 410 and that is close to the wrist (namely, an inner side of the wearable device 400), and is not connected to the wearable device 400. The airbag 420 and the device body 410 of the wearable device 400 are two separate independent structures with poor integration. In addition, as shown in FIG. 7 and FIG. 8, after the airbag 420 of the wearable device 400 expands to be an ellipsoid, an effective pressing length L of a blood vessel 210 is reduced. Consequently, precision of blood pressure detection is susceptible to disturbance of an interference signal, and is poor in stability. This affects accuracy of a detection result.

Therefore, an embodiment of this application provides an electronic device, so that the electronic device is wearable and may measure blood pressure anytime and anywhere. This helps improve accuracy of blood pressure measurement. It should be understood that the electronic device includes but is not limited to a wearable device or the like. The wearable device includes but is not limited to a watch 100 or a smartwatch.

The following further describes the electronic device in this embodiment of this application by using a watch as an example.

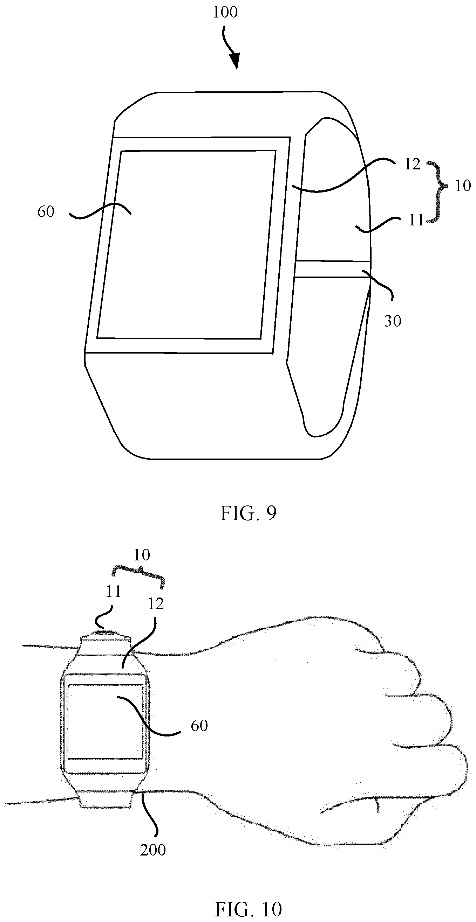

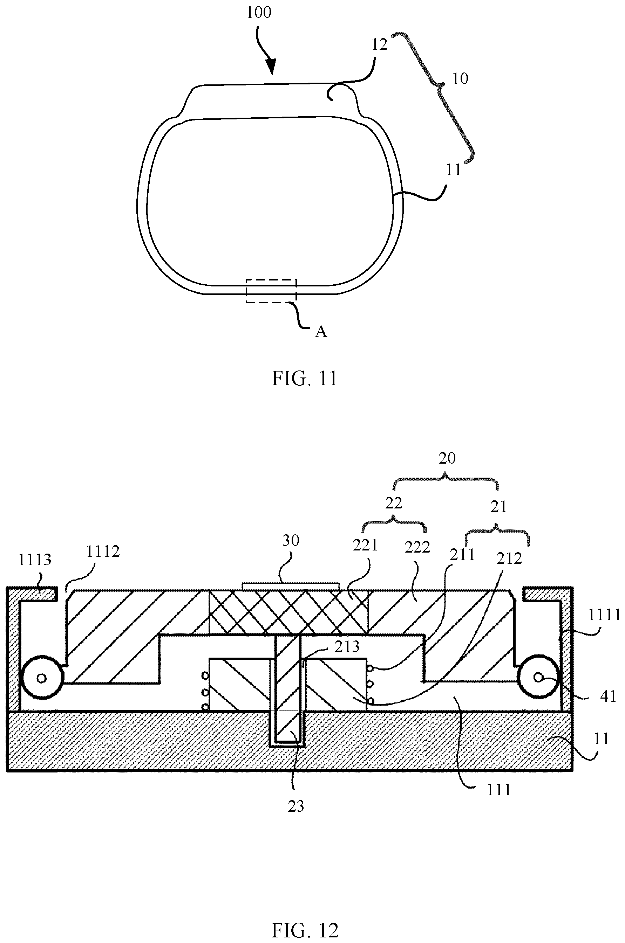

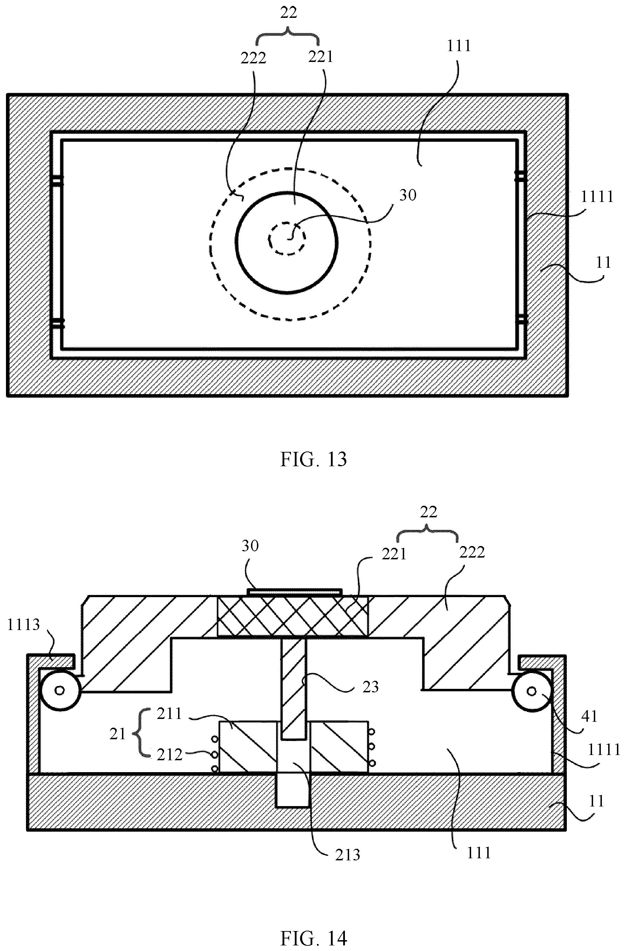

FIG. 9 is a schematic diagram of a structure of an electronic device according to an embodiment of this application. FIG. 10 is a schematic diagram of a structure of an electronic device worn on a wrist according to an embodiment of this application. FIG. 11 is a schematic diagram of a structure of an electronic device from another perspective according to an embodiment of this application. FIG. 12 is a partial sectional view that shows a part A of the electronic device in FIG. 11. FIG. 13 is a top view of the electronic device in FIG. 12. FIG. 14 is a schematic diagram of a structure of the electronic device in FIG. 12 during blood pressure measurement.

FIG. 9 and FIG. 10 show a watch. In this embodiment of this application, the watch 100 is used as an example to further describe the electronic device in this application. Refer to FIG. 10 to FIG. 14. The watch 100 may include a pressing component 20 and a pressure sensor 30, and the pressing component 20 is disposed on a wearing structure 10 of the electronic device. The pressing component 20 includes an electromagnetic driving member 21 and a pressure bearing member 22 that may move under an electromagnetic force of the electromagnetic driving member 21, and the pressure sensor 30 is disposed on the pressure bearing member 22. In this way, the pressure bearing member 22 may be fastened relative to the pressure sensor 30 while supporting the pressure sensor 30. The pressure sensor 30 is located on a surface that is of the wearing structure 10 and that faces the measurement body part 200 of a user, and is configured to press the measurement body part 200 when measuring blood pressure for the measurement body part 200. The pressure bearing member 22 is driven by the electromagnetic driving member 21 to press the pressure sensor 30 on the measurement body part 200. The pressure sensor 30 is configured to obtain a pulse wave signal of the measurement body part 200, to measure blood pressure.

It should be noted that the pulse wave signal in this embodiment is the same as the pressure wave signal mentioned in the background, and includes an oscillation wave signal and a pressure signal. The electronic device in this embodiment of this application may filter and perform fitting processing on the forging pulse wave signal to obtain an oscillation wave envelope through the oscillographic method, and estimate a blood pressure value based on a relationship between the oscillation wave envelope and blood pressure, to measure blood pressure.

When measuring blood pressure by using the oscillographic method, the electronic device in this application may measure blood pressure by applying continuously changing pressure, as shown in FIG. 3. Alternatively, the electronic device may measure blood pressure at a pressure value (for example, 100 mm Hg). It should be noted that when a pressure value is used to measure blood pressure, the pressure value needs to be greater than average arterial pressure in FIG. 5, to ensure validity and accuracy of a measurement result.

In a possible implementation, the pressure sensor 30 may transmit the obtained pulse wave signal to a processor 50 of the electronic device. After obtaining the pulse wave signal transmitted by the pressure sensor 30, the processor 50 filters and performs fitting processing on the pulse wave signal, and estimates the blood pressure value based on the relationship between the oscillation wave envelope and the blood pressure, to measure blood pressure of the measurement body part 200.

Alternatively, in another possible implementation, the pressure sensor 30 may transmit the obtained pulse wave signal to a PC end outside the electronic device. The PC end filters and performs fitting processing on the pulse wave signal, and estimates the blood pressure value based on the relationship between the oscillation wave envelope and the blood pressure, to measure blood pressure of the measurement body part 200. In this embodiment, a signal transmission manner of the pressure sensor 30 is not further limited.

Correspondingly, in this embodiment, the electronic device may alternatively be another apparatus that can be worn on a wrist. In other words, the electronic device in this application includes but is not limited to the watch 100. The watch 100 includes but is not limited to a smartwatch. When the watch 100 is a smartwatch, a processor inside the smartwatch may be used to process the pulse wave signal and calculate the blood pressure value.

The pressing component 20 may be disposed on the wearing structure 10 of the electronic device. It may be understood that the pressing component 20 may be disposed on a wearing body of the electronic device. The wearing body may include a wrist strap 11, or an electronic body 12 of the electronic device, or a wrist strap 11 and an electronic body 12 of the electronic device. Correspondingly, the watch 100 shown in FIG. 9 to FIG. 11 is used as an example. The wearing structure 10 may include the wrist strap 11 and the electronic body 12. The wrist strap 11 is connected to the electronic body 12 in a rotating manner. The electronic body 12 may be understood as a watch body of the watch 100. The pressing component 20 may be disposed in the wrist strap 11. Refer to FIG. 10 and FIG. 11. When the electronic device measures blood pressure, the pressure sensor 30 on the pressure bearing member 22 presses the measurement body part 200 (for example, an artery of the wrist) of the user.

Alternatively, the pressing component 20 may be disposed in the electronic body 12, that is, the pressing component 20 may alternatively be disposed in the watch body, and the pressure sensor 30 may press the measurement body part 200 of the user. In this case, the measurement body part 200 pressed by the pressure sensor 30 is the back of the wrist rather than an artery inside the wrist. To obtain an accurate measurement result, in actual use, the electronic body 12 of the electronic device may be rotated to the artery inside the wrist (even if the electronic body 12 is rotated to a direction opposite to that in FIG. 10 and worn on the wrist), so that the pressure sensor 30 can press the artery. In this way, a structure of the wrist strap 11 of the electronic device is not affected while blood pressure is measured.

In the following embodiments of this application, the electronic device is further described by disposing the pressing component 20 in the wrist strap 11.

Refer to FIG. 11 to FIG. 14. The pressing component 20 includes an electromagnetic driving member 21 and a pressure bearing member 22. The pressure bearing member 22 may move under an electromagnetic force of the electromagnetic driving member 21. Because the pressure sensor 30 is disposed on the pressure bearing member 22, the pressure bearing member 22 is fastened relative to the pressure sensor 30 while supporting the pressure sensor 30. In other words, the pressure sensor 30 may synchronously moves with the pressure bearing member 22 when the pressure bearing member 22 is driven by the electromagnetic driving member 21. In this way, when blood pressure needs to be measured, the electromagnetic driving member 21 may drive the pressure bearing member 22 to move in a direction towards the measurement body part 200 of the user, so as to drive the pressure sensor 30 to move in the direction towards the measurement body part 200. This implements pressing on the measurement body part. In this way, a pulse wave signal of the measurement body part 200 is obtained, so that the blood pressure is measured.

In actual application, in this embodiment, a driving force of the electromagnetic driving member 21 may be precisely controlled, so that the pressure sensor 30 presses the measurement body part 200 accurately. This helps improve accuracy of blood pressure measurement.

Specifically, in this embodiment, the pressure sensor 30 is disposed on the wearing structure 10 of the electronic device through the pressing component 20. In one aspect, real-time blood pressure measurement may be implemented, so that it is convenient for the user to monitor and manage blood pressure. In another aspect, a blood pressure measurement apparatus is greatly reduced in size than the cuff blood pressure meter 300 in the conventional technology, so that functions of an existing electronic device are more diversified.

In the conventional technology, when blood pressure is measured in an airbag pressing manner, comfort of a user is low. For example, when the cuff blood pressure meter 300 measures blood pressure, the cuff 31 is bound to an upper arm of the user, and an airbag in the cuff 31 is used to press the entire upper arm (as shown in FIG. 2). When the electronic device in this application measures blood pressure, because the electromagnetic driving member 21 drives the pressure bearing member 22 to press the measurement body part 200 by driving the pressure sensor 30, the pressure sensor 30 presses only the measurement body part 200 rather than the entire measurement body part 200 of the body. Therefore, compared with the airbag pressing manner in the conventional technology, the electronic device in this application helps improve comfort of the measurement body part 200.

Therefore, in this application, the pressing component 20 and the pressure sensor 30 are disposed on the wearing structure 10 of the electronic device. The pressing component 20 includes the electromagnetic driving member 21 and the pressure bearing member 22, and the pressure bearing member 22 may move under the electromagnetic force of the electromagnetic driving member 21. The pressure sensor 30 is disposed on the pressure bearing member 22, and is located on a surface that is of the wearing structure 10 and that faces the measurement body part 200 of the user. In this way, in one aspect, the pressure sensor 30 may synchronously move with the pressure bearing member 22, and is driven by the electromagnetic driving member 21 to press the measurement body part 200, to obtain a pulse wave signal of the measurement body part 200. This implements blood pressure measurement. In this application, the electromagnetic driving member 21 may be precisely controlled, so that the pressure sensor 30 presses the measurement body part 200 accurately. This helps improve accuracy of blood pressure measurement. In another aspect, both the pressing component 20 and the pressure sensor 30 are disposed on the wearing structure 10 of the electronic device, so that the electronic device in this application has a higher degree of integration than an existing wearable device used for blood pressure measurement, and can facilitate daily carrying and real-time blood pressure monitoring. In this way, blood pressure management is implemented for the user. In addition, compared with an existing airbag pressing manner, in this application, the pressure sensor 30 presses the measurement body part 200. This helps improve comfort of the measurement body part 200.

Refer to FIG. 11 to FIG. 14. The pressure bearing member 22 may be located on a side that is of the pressure sensor 30 and that is away from the measurement body part 200, that is, the pressure bearing member 22 is disposed towards a side that is of the pressure sensor 30 and that is away from the measurement body part 200 while carrying the pressure sensor 30. In this way, when the pressure sensor 30 is carried and fastened, and the pressure sensor 30 may be disposed close to the measurement body part 200, to shorten a distance between the pressure sensor 30 and the measurement body part 200. In this way, in one aspect, it may be convenient for the pressure sensor 30 to press the measurement body part 200, so that accuracy of blood pressure measurement is further improved. In another aspect, a movement path of the pressure bearing member 22 may be shortened, so that a requirement for a driving force of the electromagnetic driving member 21 is reduced.

Specifically, in this embodiment, when the pressure sensor 30 is pressed on the measurement body part 200 through the pressure bearing member 22, a movement direction of the pressure bearing member 22 is from the electromagnetic driving member 21 towards the measurement body part 200. It may be understood that when blood pressure does not need to be measured, the pressure bearing member 22 is disposed close to the electromagnetic driving member 21, and is located in the wearing structure 10. In other words, the pressing component 20 is disposed in the wearing structure 10 (in a state shown in FIG. 12), and the pressing component 20 is hidden through the wearing structure 10. This helps improve aesthetics of the electronic device. Correspondingly, when blood pressure needs to be measured, the pressure bearing member 22 is driven by the electromagnetic driving member 21 to drive the pressure sensor 30 to move towards the measurement body part 200. In this case, the pressure sensor 30 and the pressure bearing member 22 are located on the outside of the wearing structure 10 (in a state shown in FIG. 14), so that it is convenient for the pressure sensor 30 to press the measurement body part 200. In this way, in one aspect, when blood pressure needs to be measured, the pressure sensor 30 located on the pressure bearing member 22 and the pressure bearing member 22 move together towards the measurement body part 200, so that the measurement body part 200 is pressed. In another aspect, this improves aesthetics of the electronic device.

In this embodiment, one of the electromagnetic driving member 21 and the pressure bearing member 22 may include an electromagnet (not shown in the figure), and the other may include a magnet 221. That is, any one of the electromagnetic driving member 21 and the pressure bearing member 22 may include the electromagnet, and the other one includes the magnet 221. For example, when the electromagnetic driving member 21 includes the electromagnet, correspondingly, the pressure bearing member 22 includes the magnet 221. On the contrary, when the pressure bearing member 22 includes the electromagnet, correspondingly, the electromagnetic driving member 21 is a magnet.

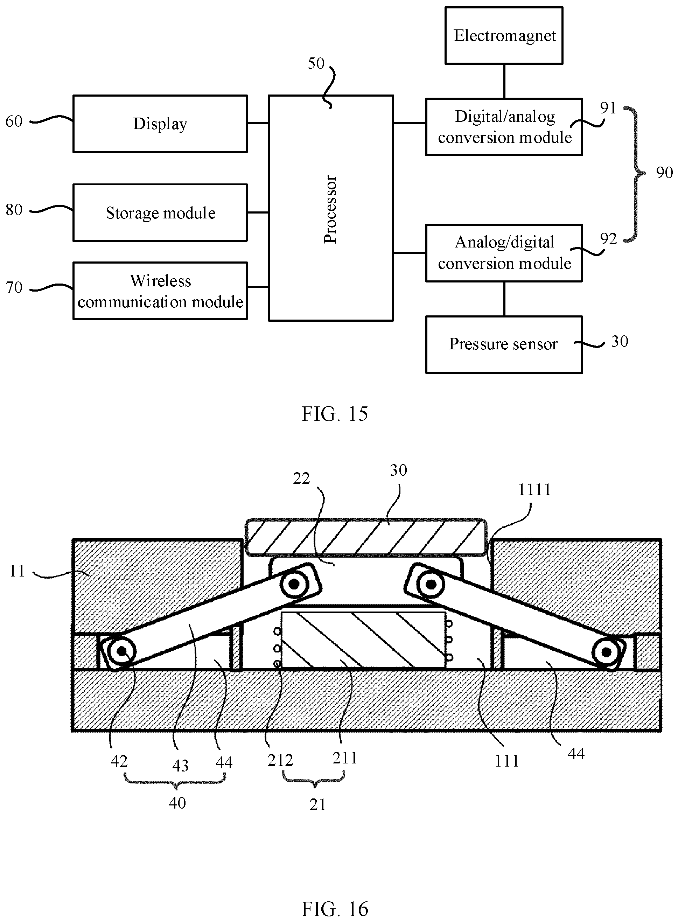

It should be noted that one of the electromagnetic driving member 21 and the pressure bearing member 22 may include the electromagnet, and the other may include the magnet. It may be understood that the electromagnet or the magnet may be a portion of the electromagnetic driving member 21 or the pressure bearing member 22, or may be an entire structure of the electromagnetic driving member 21 or the pressure bearing member 22. For example, when the pressure bearing member 22 includes the magnet 221, the magnet 221 may be a portion of the pressure bearing member 22 (as shown in FIG. 11 to FIG. 14), or may be the entire structure of the pressure bearing member 22 (as shown in FIG. 16 to FIG. 18).

For example, the magnet 221 may be a permanent magnet 221 or another magnetic module. That is, in this embodiment, the magnet 221 includes but is not limited to the permanent magnet 221.

In this embodiment, magnetic poles of the electromagnet and magnetic poles of the magnet 221 are disposed opposite to each other. In this way, a principle that like poles repel each other whereas unlike poles attract each other may be better used to drive the magnet 221 to move through the electromagnet.

Specifically, the electromagnet has an adjustable polarity, so that the magnet 221 may be driven to move by adjusting the polarity of the electromagnet. It may be understood that, when polarities of the electromagnet and the magnet 221 repel each other, the magnet 221 is driven by the electromagnet, to drive the pressure sensor 30 to move towards the measurement body part 200, and press the pressure sensor 30 on the measurement body part 200. Alternatively, when polarities of the electromagnet and the magnet 221 attract each other, the magnet 221 is driven by the electromagnet, to drive the pressure sensor 30 to move in a direction away from the measurement body part 200, so that the pressure sensor leaves the measurement body part 200 and returns to the wearing structure 10. In this way, the polarity of the electromagnet may be changed to control movement of the pressure bearing member 22 and the pressure sensor 30 relative to the electromagnetic driving member 21. In one aspect, the pressure sensor 30 may press the measurement body part 200 to obtain the pulse wave signal. When a blood pressure value is measured, a magnetic flux strength of the electromagnet may be controlled by controlling a value of an electrified current or a quantity of turns of the coil 212 of the electromagnet, so that a driving force of the electromagnet is accurately controlled, and the pressure sensor 30 presses the measurement body part 200 accurately. This helps improve accuracy of blood pressure measurement. In another aspect, the pressure sensor 30 and the pressure bearing member 22 may leave the measurement body part 200 and return to the electronic device.

For example, when the electromagnetic driving member 21 is the electromagnet, and the pressure bearing member 22 includes the magnet 221, refer to FIG. 11 to FIG. 14. When blood pressure needs to be measured, a polarity of the electromagnetic driving member 21 may be changed to make the electromagnetic driving member 21 and the magnet 221 repel each other. In this case, under an action of a repulsion force, the magnet 221 drives the pressure sensor 30 to move towards the measurement body part 200, and the pressure sensor 30 presses the measurement body part 200 to measure blood pressure. When the blood pressure is measured, the polarity of the electromagnetic driving member 21 may be changed again to make the electromagnetic driving member 21 and the magnet 221 attract each other. The magnet 221 drives, under an action of an attraction force, the pressure sensor 30 to move in a direction away from the measurement body part 200, so that the pressure sensor leaves the measurement body part 200 and returns to the wearing structure 10.

On the contrary, when the electromagnetic driving member 21 is the magnet, and when the pressure bearing member 22 is the electromagnet, movement is relative. Therefore, when blood pressure needs to be measured, the polarity of the pressure bearing member 22 may be changed to make the pressure bearing member 22 and the magnet 221 repel each other, so that under an action of a repulsion force, the pressure bearing member 22 drives the pressure sensor 30 to move towards the measurement body part 200, and the pressure sensor 30 presses the measurement body part 200 to measure the blood pressure. When the blood pressure is measured, the polarity of the pressure bearing member 22 may be changed again to make the pressure bearing member 22 and the magnet attract each other. The pressure bearing member 22 drives, under an action of an attraction force, the pressure sensor 30 to move in a direction away from the measurement body part 200, so that the pressure sensor leaves the measurement body part 200 and returns to the wearing structure 10.

It should be understood that, in actual use, the polarity of the electromagnet may be adjustable by changing a direction of the electrified current in the electromagnet.

Because the electromagnet has magnetism after being powered on, and the magnetism disappears after the electromagnet is powered off, in an actual application, the electromagnet is powered on when blood pressure needs to be measured or in a process in which the pressure sensor 30 returns. Sometimes, the electromagnet is not powered on in other time (when blood pressure does not need to be measured) alternatively, so that displacement movement of the magnet 221 relative to the electromagnet may be avoided while energy consumption of the electronic device is reduced. This helps improve stability of the electronic device.

Alternatively, in a possible implementation, to return the pressure bearing member 22 and the pressure sensor 30, a clamping structure (for example, a pressing cover) in the conventional technology may alternatively be used to force the pressure bearing member 22 and the pressure sensor 30 to return to the wearing structure 10.

In this embodiment, which one of the electromagnetic driving member 21 and the pressure bearing member 22 is the electromagnet or the magnet is not further limited.

In the following embodiment, the electronic device is further described by using an example in which the electromagnetic driving member 21 is the electromagnet and the pressure bearing member 22 includes the magnet 221.

Specifically, in this embodiment, the pressure bearing member 22 is fixedly connected to the pressure sensor 30 or is detachably connected to the pressure sensor 30. In this way, the pressure sensor 30 may be fastened to the pressure bearing member 22, and the pressure bearing member 22 supports the pressure sensor 30. In one aspect, when the pressure bearing member 22 is driven by the electromagnetic driving member 21 to move relative to the electromagnetic driving member 21, the pressure sensor 30 may synchronously move with the pressure bearing member 22. In another aspect, compared with an existing wearable device 400 having a blood pressure measurement function, the electronic device may be enhanced in integrated structure.

For example, the pressure bearing member 22 may be connected to the pressure sensor 30 through a fastener, a threaded connection, or a buckle connection. This can facilitate removing and mounting of the pressure bearing member 22 or the pressure sensor 30.

The electromagnetic driving member 21 may also be fastened or detachably connected to the mounting cavity 111, so that the electromagnetic driving member 21 is fastened relative to the mounting cavity 111. The electromagnetic driving member 21 may be connected to the mounting cavity 111 through a fastener, a threaded connection, or a buckle connection. This can facilitate removing and mounting of the electromagnetic driving member 21.

In this embodiment, the electromagnet may include a magnetic core 211 and a coil 212 wound around an outer peripheral side of the magnetic core 211. In this way, when a current flows into the coil 212, a magnetic field is generated around the coil 212. The magnetic field is used to drive the pressure bearing member 22 and the pressure sensor 30 to move relative to the measurement body part 200, so as to measure blood pressure.

It should be understood that the coil 212 wound around the outer peripheral side of the magnetic core 211 may be understood as that the coil 212 is wound outside the magnetic core 211 in a peripheral direction (as shown in FIG. 11).

In an actual application, a magnitude of the electrified current may be controlled to perform precise stepless control on the magnetic flux strength of the electromagnet, so that the pressure sensor 30 presses the measurement body part 200 accurately. This helps improve accuracy of blood pressure measurement. The stepless control may be understood as a smooth adjustment manner. In this embodiment, the stepless control on the electromagnet mainly refers to controlling a magnitude of the magnetic flux strength of the electromagnet within a range, and performing smooth adjustment on the magnetic flux strength instead of jumping adjustment.

In some embodiments, magnetic flux strength of an electromagnetic field may be precisely adjusted, for example, through stepless adjustment, so that the pressure sensor 30 presses the measurement body part 200 accurately. This helps improve accuracy of blood pressure measurement. Therefore, compared with a blood pressure measurement apparatus in the conventional technology, the electronic device in this embodiment of this application may not only press the measurement body part 200 accurately, but also measure blood pressure of an artery of a wrist, so that blood pressure measurement may be more accurate, and a medical requirement may be met.

Refer to FIG. 11 to FIG. 14. The mounting cavity 111 is disposed on a surface that is in the wrist strap 11 and that faces the measurement body part 200. In this way, it is convenient to mount and fasten the pressing component 20 and the pressure sensor 30, and the pressing component 20 and the pressure sensor 30 may be disposed close to the measurement body part 200. This facilitates pressing of the measurement body part 200 and blood pressure measurement.

That at least a portion of the pressing component 20 is disposed in the mounting cavity 111 may be understood as that the pressing component 20 may be totally disposed in the mounting cavity 111 (as shown in FIG. 12), or the pressing component 20 may be partially disposed in the mounting cavity 111 (as shown in FIG. 14). In this embodiment of this application, the pressure bearing member 22 in the pressing component 20 is a structure that moves relative to the electromagnetic driving member 21 during blood pressure measurement. Therefore, a position relationship between the pressing component 20 and the mounting cavity 111 is not limited in this embodiment. In actual use, to improve aesthetics of the electronic device, when no blood pressure test is performed, the pressing component 20 is totally disposed in the mounting cavity 111.

To facilitate movement of the pressure bearing member 22, the pressure bearing member 22 is slidably connected to an inner wall 1111 of the mounting cavity 111. In this way, when the pressure bearing member 22 is mounted in the mounting cavity 111, it may be convenient for the pressure bearing member 22 to be driven by the electromagnetic driving member 21 to move. This reduces abrasion caused to the pressure bearing member 22 and prolongs a service life of the electronic device.

The electronic device further includes at least one sliding component 40, and the sliding component 40 is connected between the pressure bearing member 22 and the inner wall 1111 of the mounting cavity 111, so that the pressure bearing member 22 is slidably connected to the inner wall 1111 of the mounting cavity 111. In this way, when the pressure bearing member 22 is slidably connected to the inner wall 1111 of the mounting cavity 111 of the wrist strap 11, abrasion caused to the pressure bearing member 22 or the mounting cavity 111 when the pressure bearing member 22 moves in the mounting cavity 111 may be reduced, so as to prolong the service life of the electronic device.

It should be understood that the electronic device further includes at least one sliding component 40, that is, the electronic device may include one sliding component 40, or may include a plurality of sliding components 40. In this embodiment, a quantity of sliding components 40 is not further limited, provided that the pressure bearing member 22 may be slidably connected to the inner wall 1111 of the mounting cavity 111 through the sliding component 40.

For example, there may be a plurality of sliding components 40, and the sliding components 40 are disposed in different directions on a peripheral side of the pressure bearing member 22. For example, there may be four sliding components 40, and the four sliding components 40 may be symmetrically disposed on two sides of the pressure bearing member 22. In this way, in one aspect, sliding between the pressure bearing member 22 and the mounting cavity 111 may be more stable, and stability performance of the pressing component 20 in the wrist strap 11 may be enhanced. This helps improve a pressing effect of the pressure sensor 30. In another aspect, connection manners of the pressure bearing member 22 and the mounting cavity 111 may be more diversified.

In this embodiment, the sliding component 40 may be a pulley component. Alternatively, the sliding component 40 may be a guide rail component. Alternatively, the sliding component 40 may be a sliding structure in another form. In this embodiment, a structure form of the sliding component 40 is not further limited. In this embodiment, provided that the sliding component 40 may be connected between the pressure bearing member 22 and the inner wall 1111 of the mounting cavity 111, the pressure bearing member 22 is slidbaly connected to the inner wall 1111 of the mounting cavity 111.

In the following embodiments of this application, the electronic device in this application is further described separately by using the pulley component as an application scenario 1 and the guide rail component as an application scenario 2.

### Scenario 1

As shown in FIG. 11 to FIG. 14, in a possible implementation, in this application scenario, each sliding component 40 may include a first pulley 41 disposed on a side wall of the pressure bearing member 22 and a slide rail (not shown in the figure) disposed on the inner wall 1111 of the mounting cavity 111. The first pulley 41 and the slide rail are disposed opposite to each other, and the first pulley 41 may slide along the slide rail, so that the pressure bearing member 22 is slidbaly connected to the inner wall 1111 of the mounting cavity 111. The first pulley 41 and the slide rail form the foregoing pulley component. In this way, the sliding between the pressure bearing member 22 and the mounting cavity 111 may be smoother, and the pressure bearing member 22 may slide according to a set track of the slide rail.

Refer to FIG. 12 to FIG. 14. The pressure bearing member 22 further includes a lifting body, and the magnet 221 is embedded in a lifting platform 222 and is fastened relative to the magnet 221. The pulley component is disposed on a peripheral side of the lifting platform 222. That is, the first pulley 41 is disposed on a side wall of the lifting platform 222, so that the first pulley 41 may be driven by the electromagnetic driving member 21 to slide in the slide rail. In this way, the lifting platform 222 and the magnet 221 may be slidably connected on the inner wall 1111 of the mounting cavity 111, to move towards the measurement body part 200 or away from the measurement body part 200.

For example, refer to FIG. 12 to FIG. 14. In this embodiment, there may be four or two first pulleys 41, and the four or two first pulleys 41 are symmetrically distributed on two sides of the pressure bearing member 22, so that sliding between the pressure bearing member 22 and the mounting cavity 111 is smoother, and connection stability between the pressure bearing member 22 and the mounting cavity 111 may be improved.

To limit the first pulley 41, refer to FIG. 12 and FIG. 14, a limiting part 1113 is disposed at an opening 1112 of the mounting cavity 111, and the limiting part 1113 is correspondingly disposed outside the first pulley 41 in a pressing direction. In other words, the limiting part 1113 is disposed corresponding to the first pulley, and is located on a side that is of the first pulley and that faces the measurement body part 200. In this way, in one aspect, a movable stroke of the pressure bearing member 22 may be limited through the limiting part 1113. In another aspect, the first pulley 41 may be shielded, so as to enhance aesthetics and sealing performance of the electronic device.

The limiting part 1113 and the wrist strap 11 are of an integrated structure, that is, the limiting part 1113 is formed on the wrist strap 11. Alternatively, the limiting part 1113 may be connected to the wrist strap 11 through clamping or another detachable connection. The limiting part 1113 is detachably connected to the wrist strap 11, so as to facilitate mounting or removing of the pressing component 20. In this embodiment, whether the limiting part 1113 and the wrist strap 11 are of an integrated structure is not further limited.

In a possible implementation, the limiting part 1113 may be a plate-like structure that can shield the first pulley 41. An area that is in the mounting cavity 111 and that is opposite to the limiting part 1113 forms a mounting and sliding area of the first pulley 41. Correspondingly, the slide rail is disposed on an inner wall 1111 of the mounting and sliding area in the mounting cavity 111.

It should be understood that, in this embodiment, the opening 1112 of the mounting cavity 111 is formed on a side that is of the mounting cavity 111 and that faces the measurement body part 200. A size of the mounting cavity 111 should adapt to a size of the pressing component 20. In this embodiment, a size and a shape of an accommodating cavity are not further limited.

To further guide the movable stroke of the pressure bearing member 22, in this embodiment, a guide member 23 (as shown in FIG. 12 and FIG. 14) is further disposed between the pressure bearing member 22 and the electromagnetic driving member 21, a first guide groove 213 corresponding to the guide member 23 is further disposed in the electromagnetic driving member 21, a first end of the guide member 23 is connected to the magnet 221, and a second end of the guide member 23 passes through the first guide groove 213 and may move in an extension direction of the first guide groove 213. In this way, the guide member 23 slides in the first guide groove 213, so that movement of the pressure bearing member 22 may be better guided.

The extension direction of the first guide groove 213 may be understood as an extension (as shown in FIG. 12 and FIG. 14) of the first guide groove 213 to the wrist strap 11 on a side that is of the electromagnetic driving member 21 and that is away from the measurement body part 200. In this way, a guide range of the guide member 23 for the pressure bearing member 22 may be expanded by using the extension direction of the first guide groove 213. To improve assembly stability of the pressing component 20 in the wrist strap 11, the wrist strap 11 may be a metal wrist strap, a fluoro rubber wrist strap, or another wrist strap made of a material that is not easy to deform with the pressing component 20. In this way, deformation of the wrist strap 11 relative to the pressing component 20 or the pressure sensor 30 may be avoided, so that the pressing component 20 is more securely connected in the wrist strap 11. This helps improve stability performance of the electronic device.

In a possible implementation, when the electronic device in this application does not measure blood pressure, the pressure sensor 30 may be even with a surface of the wearing structure 10, that is, a surface that is of the pressure sensor 30 and that is close to the measurement body part 200 and a surface that is of the wearing structure 10 and that is close to the measurement body part 200 are in a same plane. In this case, the pressure bearing member 22 is totally located in the wearing structure 10. Alternatively, the pressure sensor 30 may protrude from a surface (as shown in FIG. 12) of the wearing structure 10 (for example, the wrist strap 11). To be specific, a surface that is of the pressure sensor 30 and that is close to the measurement body part 200 is closer to the measurement body part 200 than a surface that is of the wearing structure 10 and that is close to the measurement body part 200, so that at least a portion of the pressure sensor 30 is exposed inside the wrist strap 11. In this way, when the pressure sensor 30 presses the measurement body part 200, the pressure sensor 30 may be disposed in a more diversified manner. This helps improve diversity of the electronic device.

FIG. 15 is a principle block diagram of blood pressure measurement performed by an electronic device according to an embodiment of this application.

Refer to FIG. 15. In this embodiment, the electronic device further includes a processor 50. The processor 50 is electrically connected to the pressure sensor 30. The processor 50 is configured to filter and perform fitting processing on an obtained pulse wave signal, and measure a blood pressure value of a user based on the obtained pulse wave signal. In this way, blood pressure of the user may be measured through the processor 50 and the pressure sensor 30, so that the user monitors the blood pressure in real time and manages the blood pressure.

In this embodiment, the processor 50 may be an electronic device such as a processor 50 in a smartwatch, and the processor 50 processes a pulse wave signal and calculates a blood pressure value. For example, the processor 50 may be a microcontroller unit (microcontroller unit, MCU).

In a possible implementation, in this embodiment, a signal conversion module 90 in the processor 50 may be used to perform signal conversion processing on the pulse wave signal obtained by the pressure sensor 30, and then transmit a converted pulse wave signal to the processor 50, or convert a control signal of the processor 50, and then transmit a converted control signal to an electromagnet. Alternatively, refer to FIG. 15. In this embodiment, a signal conversion module 90 may be additionally disposed to perform signal conversion alternatively. The signal conversion module 90 includes a digital/analog conversion module 91 and an analog/digital conversion module 92. The electromagnet is electrically connected to the processor 50 through the digital/analog conversion module 91. The digital/analog conversion module 91 is configured to convert a digital control signal sent by the processor 50 into an analog signal, so as to control whether the electromagnet is powered on and a direction of a current. In this way, existence of a magnetic field of the electromagnet and a polarity of the electromagnet are controlled. The pressure sensor 30 is electrically connected to the processor 50 through the analog-to-digital conversion module 92. The analog-to-digital conversion module 92 is configured to obtain a pulse wave signal of the pressure sensor 30, convert the pulse wave signal into a digital signal, and transmit the digital signal to the processor 50. The processor 50 may control, based on a magnitude of the pulse wave signal by controlling the polarity of the electromagnet, whether the pressure bearing member 22 continues to drive the pressure sensor 30 to press the measurement body part 200. For example, when a pressure value of the pulse wave signal obtained by the pressure sensor 30 reaches a preset upper limit of the pressure value, the processor 50 may control the polarity of the electromagnet (that is, reverse electrification) by controlling a direction of an electrified current, so that the electromagnet and the polarity of the magnet 221 magnetically attract each other. In this case, under an action of an attraction force, the pressure bearing member 22 may drive the pressure sensor 30 to gradually move towards a side away from the measurement body part 200, so that pressure of the pressure sensor 30 on the measurement body part 200 gradually decreases. When the pressure sensor 30 and the pressure bearing member 22 are fastened, the pressure sensor 30 and the pressure bearing member 22 may return gradually.

To help the user directly obtain the blood pressure value, refer to FIG. 15. The electronic device further includes a display 60. The display 60 is electrically connected to the processor 50, and is configured to display the blood pressure value. In this way, the measured blood pressure value may be intuitively displayed through the display 60, so that it is convenient for the user to obtain the blood pressure value.

The display 60 may be a display 60 on an electronic device body. For example, when the electronic device is a smartwatch, the display 60 that is on the electronic device and that is configured to display a blood pressure value may be a display 60 of the smartwatch. In this way, the user can intuitively observe the measured blood pressure value directly through the display 60 of the smartwatch.

Specifically, in this embodiment, a storage module 80 may be disposed in the electronic device. The storage module 80 is electrically connected to the processor 50 and the pressure sensor 30, and may be configured to store a measured blood pressure value, and may store a database used for blood pressure calculation. The processor 50 may calculate the blood pressure value according to an algorithm in the database in the storage module 80.

Further, the electronic device further includes a wireless communication module 70, and the wireless communication module 70 is electrically connected to the processor 50. In this way, communication with the wearing structure 10 of the electronic device or an external device may be implemented through the wireless communication module 70. Therefore, functions of the electronic device are more diversified.

It should be understood that the electronic device further includes a power supply (not shown in the figure) configured to supply power. The power supply is electrically connected to a power consuming module, for example, the power supply is electrically connected to the processor 50, the display 60, the wireless communication module 70, the signal conversion module 90, the electromagnet, and the like. The power supply may be a power supply of the electronic device, or may be an additional power supply disposed in the electronic device such as a wrist strap 11 or an electronic body 12.

The electronic device in this embodiment of this application includes a wearing structure 10, a pressing component 20, and a pressure sensor 30. The pressing component 20 is disposed on the wearing structure 10, and the pressure sensor 30 is disposed on the pressing component 20. In this way, in one aspect, the pressure sensor 30 may press the measurement body part 200, so that the electronic device is wearable and has a blood pressure measurement function. In another aspect, the pressure sensor 30 may be assembled on the wearing structure 10 through the pressing component 20. This helps improve an integrated structure of the electronic device.

It should be noted that, because a mounting cavity 111 is disposed in the wrist strap 11, a gap inevitably exists between the pressure bearing member 22 and the mounting cavity 111. To improve sealing performance of the electronic device such as a watch 100, an elastic sealing member (not shown in the figure) may be connected between the pressure bearing member 22 and the mounting cavity 111. This ensures that the electronic device has specific sealing performance while implementing blood pressure measurement. For example, the elastic member includes but is not limited to silica gel.

In this application, the pressing component and the pressure sensor are disposed on the wearing structure of the electronic device. The pressing component includes an electromagnetic driving member and the pressure bearing member, and the pressure bearing member may move under an electromagnetic force of the electromagnetic driving member. The pressure sensor is disposed on the pressure bearing member, and is located on a surface that is of the wearing structure and that faces the measurement body part of the user. In this way, in one aspect, the pressure sensor 30 may be driven by the electromagnetic driving member to press the measurement body part, to obtain a pulse wave signal of the measurement body part. This implements blood pressure measurement. In another aspect, the pressing component and the pressure sensor are both disposed on the wearing structure of the electronic device, so that an integration degree of the electronic device in this application is high.

### Scenario 2

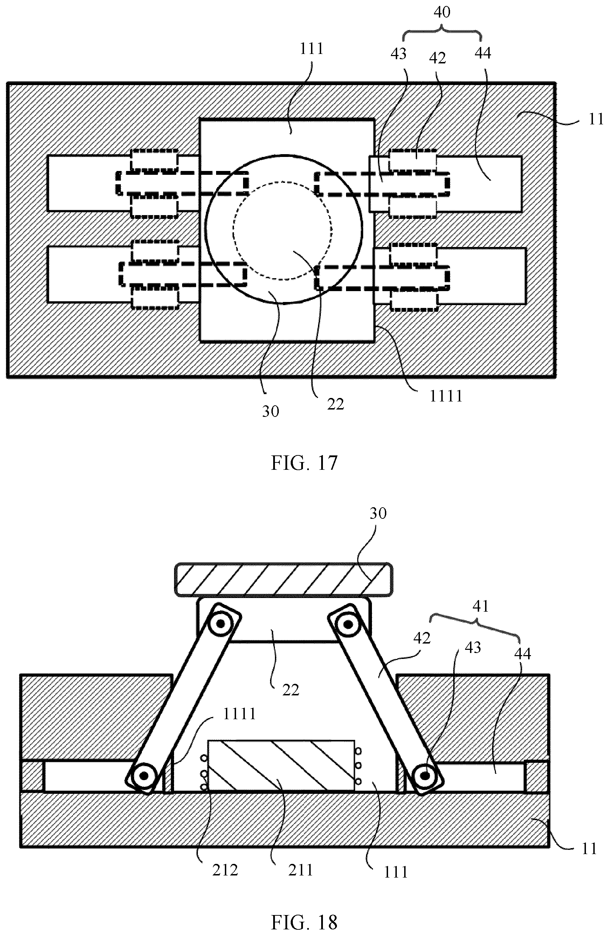

In addition, this embodiment also provides an electronic device of another structure. FIG. 16 is a schematic diagram of another structure of the electronic device in FIG. 12 according to an embodiment of this application. FIG. 17 is a top view of the electronic device in FIG. 16 according to an embodiment of this application. FIG. 18 is a schematic diagram of a structure of the electronic device in FIG. 16 according to an embodiment of this application during blood pressure measurement.

On the basis of the foregoing, a difference between Scenario 2 and the foregoing Scenario 1 lies in that the sliding component 40 in this embodiment is a guide rail component. The pressure bearing member 22 in this scenario is a magnet 221. The sliding component 40 includes a second pulley 42, a connection arm 43, and a second guide groove 44. The second pulley 42, the connection arm 43, and the second guide groove 44 form the guide rail component. It should be noted that, for ease of describing a difference of the sliding component 40, FIG. 16 to FIG. 18 mainly show a main structure of the electronic device including the sliding component 40, and a structure such as the wrist strap 11 is not shown in the figure.

The second guide groove 44 and the mounting cavity 111 are fastened relative to each other, that is, the second guide groove 44 and the mounting cavity 111 do not move relative to each other. An extension direction of the second guide groove 44 is mutually staggered with a pressing direction of the electromagnetic driving member 21. In other words, the extension direction of the second guide groove 44 is mutually crossed with the pressing direction of the electromagnetic driving member 21.

For example, when the extension direction of the second guide groove 44 is parallel to a length direction of the wrist strap 11 (namely, the horizontal direction in FIG. 16 and FIG. 18), the pressing direction of the electromagnetic driving member 21 may be a direction perpendicular to the length direction of the wrist strap 11, namely, a width direction of the wrist strap 11. A first end of the connection arm 43 is hinged with the pressure bearing member 22, and the second pulley 42 is disposed at a second end of the connection arm 43. The second pulley 42 may slide in the guide groove, and the second pulley 42 is hinged with the second end of the connection arm 43, so that the connection arm 43 drives the pressure bearing member 22 to move in the pressing direction. In this way, sliding between the pressure bearing member 22 and the mounting cavity 111 may be smoother through the sliding component 40, and the moveable stroke of the pressure bearing member 22 may be limited through the sliding component 40.

When blood pressure needs to be measured, driven by the electromagnetic driving member 21, the pressure bearing member 22, along with the connection arm 43, moves towards the measurement body part 200 of the user (that is, move towards the top in FIG. 18), and presses the measurement body part 200. On the contrary, when an upper limit of pressure in a pulse wave signal is reached or the pressure bearing member 22 needs to return, driven by the electromagnetic driving member 21 or pressed by another external force, the pressure bearing member 22 moves, along with the connection arm 43, in a direction away from the measurement body part 200 of the user, that is, moves towards the bottom in FIG. 18, until moving to a state shown in FIG. 16, and the pressure bearing member 22 returns to the wrist strap 11.

For example, refer to FIG. 16 to FIG. 18. In this embodiment, there may be four or two sliding components 40, and the four or two sliding components 40 are symmetrically distributed on two sides of the pressure bearing member 22, so that sliding between the pressure bearing member 22 and the mounting cavity 111 is smoother, and connection stability between the pressure bearing member 22 and the mounting cavity 111 may be improved.

It should be understood that when the pressure bearing member 22 moves, the connection arm 43 moves in the second guide groove 44 through the second pulley 42. Therefore, a length of the connection arm 43 and a length of the second guide groove 44 may be adjusted to limit a movable stroke of the pressure bearing member 22.

In a possible implementation, the first end of the connection arm 43 may be hinged with the pressure bearing member 22 through the second pulley 42, that is, the second pulley 42 is fastened on a side wall of the pressure bearing member 22, and the first end of the connection arm 43 is hinged with the second pulley.

For example, the second guide groove 44 may be located in the mounting cavity 111. In this case, the connection arm 43 passes through the mounting cavity 111. Alternatively, the second guide groove 44 may be formed as a cavity (not shown in the figure) in communication with the mounting cavity 111. In this way, the sliding component 40 and the electronic device may be disposed in more diversified manners when the second pulley 42 slides in the second guide groove 44. When the cavity is formed in the wrist strap 11, the second guide groove 44 is also located in the wrist strap 11. In this case, the connection arm 43 may pass through the mounting cavity 111 and the cavity (as shown in FIG. 16 to FIG. 18).

Alternatively, in a possible implementation, when the second guide groove 44 is located on an outer sidewall outside the wrist strap 11, the connection arm 43 may also be located on the outer sidewall of the wrist strap 11 and located outside the mounting cavity 111.

In this application, the pressing component and the pressure sensor are disposed on the wearing structure of the electronic device. The pressing component includes the electromagnetic driving member and the pressure bearing member, and the pressure bearing member may move under the electromagnetic force of the electromagnetic driving member. The pressure sensor is disposed on the pressure bearing member, and is located on a surface that is of the wearing structure and that faces the measurement body part of the user. In this way, in one aspect, the pressure sensor may be driven by the electromagnetic driving member to press the measurement body part, to obtain a pulse wave signal of the measurement body part. This implements blood pressure measurement. In another aspect, the pressing component and the pressure sensor are both disposed on the wearing structure of the electronic device, so that an integration degree of the electronic device in this application is high.

## Claims

1. A wearable electronic device (100), comprising:
a pressing component (20);
a pressure sensor (30);
wherein the pressing component (20) comprises an electromagnetic driving component (21) and a pressure bearing component (22); wherein the pressure bearing component (22) may move under an electromagnetic force of the electromagnetic driving component (21);
the pressure sensor (30) is disposed on the pressure bearing component (22), and the pressure sensor (30) is located on a surface that is of the pressure bearing component (22) and that faces a measurement body part of a user;
the pressure bearing component (22) is configured to be driven by the electromagnetic driving component (21) to press the pressure sensor (30) on the measurement body part, and
the pressure sensor (30) is configured to detect a pulse wave signal of the measurement body part,
wherein the electronic device comprises a wrist strap (11) and an electronic body (12), the wrist strap (11) is connected to the electronic body (12), and the pressing component (20) is disposed in the wrist strap (11),
wherein a mounting cavity is disposed on a surface that is in the wrist strap (11) and that faces the measurement body part, and at least a portion of the pressing component (20) is disposed in the mounting cavity (111),
wherein the pressure bearing component (22) is slidably connected to an inner wall of the mounting cavity (111).

2. The electronic device according to claim 1, wherein when the pressure sensor (30) is pressed on the measurement body part by the pressure bearing component (22), a movement direction of the pressure bearing component (22) is from the electromagnetic driving component (21) towards the measurement body part.

3. The electronic device according to claim 1 or 2, wherein one of the electromagnetic driving component (21) and the pressure bearing component (22) comprises an electromagnet, and the other comprises a magnet; and magnetic poles of the electromagnet and magnetic poles of the magnet are disposed opposite to each other; and
the electromagnet has an adjustable polarity, so that when poles of the electromagnet and poles of the magnet repel each other, the pressure sensor (30) is pressed on the measurement body part, or when poles of the electromagnet and poles of the magnet attract each other, the pressure sensor (30) leaves the measurement body part.

4. The electronic device according to claim 3, wherein the pressure bearing component (22) is fixedly connected to the pressure sensor (30) or is detachably connected to the pressure sensor (30).

5. The electronic device according to claim 3 or 4, wherein the electromagnet comprises a magnetic core and a coil wound around an outer peripheral side of the magnetic core.

6. The electronic device according to any one of claims 1-5, further comprising at least one sliding component, wherein the sliding component is connected between the pressure bearing component (22) and the inner wall of the mounting cavity (111), so that the pressure bearing component (22) is slidably connected to the inner wall of the mounting cavity (111).

7. The electronic device according to claim 6, wherein each sliding component comprises a first pulley disposed on a side wall of the pressure bearing component (22) and a slide rail disposed on the inner wall of the mounting cavity (111), the pulley and the slide rail are disposed opposite to each other, and the first pulley can slide along the slide rail, so that the pressure bearing component (22) is slidably connected to the inner wall of the mounting cavity (111).

8. The electronic device according to claim 7, wherein a limiting part is disposed at an opening of the mounting cavity (111), and the limiting part is correspondingly disposed outside the first pulley in a pressing direction.

9. The electronic device according to claim 7 or 8 and according to claim 3, wherein a guide component is further disposed between the pressure bearing component (22) and the electromagnetic driving component (21), a first guide groove corresponding to the guide component is further disposed in the electromagnetic driving component (21), a first end of the guide component is connected to the magnet, and a second end of the guide component passes through the first guide groove and may move in an extension direction of the first guide groove.

10. The electronic device according to claim 8, wherein the sliding component comprises a second pulley, a connection arm, and a second guide groove, the second guide groove and the mounting cavity (111) are fastened relative to each other, and an extension direction of the second guide groove is staggered with a pressing direction of the electromagnetic driving component (21); a first end of the connection arm is hinged with the pressure bearing component (22), the second pulley is disposed at a second end of the connection arm, and the second pulley can slide in the guide groove, so that the connection arm drives the pressure bearing component (22) to move in the pressing direction.

11. The electronic device according to claim 10, wherein the second guide groove is located in the mounting cavity (111); or
the second guide groove is formed into a cavity in communication with the mounting cavity (111).

12. The electronic device according to any one of claims 1 to 11, wherein the wrist strap (11) is a metal wrist strap or a fluoro rubber wrist strap.

## Patentansprüche

1. Tragbare elektronische Vorrichtung (100), die umfasst:
eine Drückkomponente (20);
einen Drucksensor (30);
wobei die Drückkomponente (20) eine elektromagnetische Antriebskomponente (21) und eine Drucklagerkomponente (22) umfasst; wobei sich die Drucklagerkomponente (22) unter einer elektromagnetischen Kraft der elektromagnetischen Antriebskomponente (21) bewegen kann;
der Drucksensor (30) auf der Drucklagerkomponente (22) angeordnet ist und sich der Drucksensor (30) auf einer Oberfläche, die zu der Drucklagerkomponente (22) gehört und die einem Messkörperteil eines Benutzers zugewandt ist, befindet;
die Drucklagerkomponente (22) konfiguriert ist, um durch die elektromagnetische Antriebskomponente (21) angetrieben zu werden, um den Drucksensor (30) auf den Messkörperteil zu drücken, und
der Drucksensor (30) konfiguriert ist, um ein Pulswellensignal des Messkörperteils zu erfassen,
wobei die elektronische Vorrichtung ein Armband (11) und einen elektronischen Körper (12) umfasst, das Armband (11) mit dem elektronischen Körper (12) verbunden ist und die Drückkomponente (20) in dem Armband (11) angeordnet ist, wobei ein Montagehohlraum auf einer Oberfläche, die in dem Armband (11) ist und die dem Messkörperteil zugewandt ist, angeordnet ist und mindestens ein Abschnitt der Drückkomponente (20) in dem Montagehohlraum (111) angeordnet ist,
wobei die Drucklagerkomponente (22) mit einer Innenwand des Montagehohlraums (111) gleitbar verbunden ist.

2. Elektronische Vorrichtung nach Anspruch 1, wobei, wenn der Drucksensor (30) durch die Drucklagerkomponente (22) auf den Messkörperteil gedrückt wird, eine Bewegungsrichtung der Drucklagerkomponente (22) von der elektromagnetischen Antriebskomponente (21) zu dem Messkörperteil hin ist.

3. Elektronische Vorrichtung nach Anspruch 1 oder 2, wobei eine der elektromagnetischen Antriebskomponente (21) und der Drucklagerkomponente (22) einen Elektromagneten umfasst und die andere einen Magneten umfasst; und Magnetpole des Elektromagneten und Magnetpole des Magneten einander gegenüberliegend angeordnet sind; und der Elektromagnet eine anpassbare Polarität aufweist, sodass, wenn Pole des Elektromagneten und Pole des Magneten sich gegenseitig abstoßen, der Drucksensor (30) auf den Messkörperteil gedrückt wird, oder wenn Pole des Elektromagneten und Pole des Magneten sich gegenseitig anziehen, der Drucksensor (30) den Messkörperteil verlässt.

4. Elektronische Vorrichtung nach Anspruch 3, wobei die Drucklagerkomponente (22) mit dem Drucksensor (30) fest verbunden ist oder mit dem Drucksensor (30) lösbar verbunden ist.

5. Elektronische Vorrichtung nach Anspruch 3 oder 4, wobei der Elektromagnet einen Magnetkern und eine Spule, die um eine äußere Umfangsseite des Magnetkerns herum gewickelt ist, umfasst.

6. Elektronische Vorrichtung nach einem der Ansprüche 1 bis 5, die ferner mindestens eine Gleitkomponente umfasst, wobei die Gleitkomponente zwischen der Drucklagerkomponente (22) und der Innenwand des Montagehohlraums (111) verbunden ist, sodass die Drucklagerkomponente (22) mit der Innenwand des Montagehohlraums (111) gleitbar verbunden ist.

7. Elektronische Vorrichtung nach Anspruch 6, wobei jede Gleitkomponente eine erste Rolle, die an einer Seitenwand der Drucklagerkomponente (22) angeordnet ist, und eine Gleitschiene, die an der Innenwand des Montagehohlraums (111) angeordnet ist, umfasst, wobei die Rolle und die Gleitschiene einander gegenüberliegend angeordnet sind und die erste Rolle entlang der Gleitschiene gleiten kann, sodass die Drucklagerkomponente (22) mit der Innenwand des Montagehohlraums (111) gleitbar verbunden ist.

8. Elektronische Vorrichtung nach Anspruch 7, wobei ein Begrenzungsteil an einer Öffnung des Montagehohlraums (111) angeordnet ist und der Begrenzungsteil in einer Drückrichtung außerhalb der ersten Rolle entsprechend angeordnet ist.

9. Elektronische Vorrichtung nach Anspruch 7 oder 8 und nach Anspruch 3, wobei eine Führungskomponente ferner zwischen der Drucklagerkomponente (22) und der elektromagnetischen Antriebskomponente (21) angeordnet ist, eine erste Führungsnut, die der Führungskomponente entspricht, ferner in der elektromagnetischen Antriebskomponente (21) angeordnet ist, ein erstes Ende der Führungskomponente mit dem Magneten verbunden ist und ein zweites Ende der Führungskomponente durch die erste Führungsnut verläuft und sich in einer Erstreckungsrichtung der ersten Führungsnut bewegen kann.

10. Elektronische Vorrichtung nach Anspruch 8, wobei die Gleitkomponente eine zweite Rolle, einen Verbindungsarm und eine zweite Führungsnut umfasst, die zweite Führungsnut und der Montagehohlraum (111) relativ zueinander befestigt sind und eine Erstreckungsrichtung der zweiten Führungsnut mit einer Drückrichtung der elektromagnetischen Antriebskomponente (21) versetzt ist; ein erstes Ende des Verbindungsarms an die Drucklagerkomponente (22) gelenkig angeschlossen ist, die zweite Rolle an einem zweiten Ende des Verbindungsarms angeordnet ist und die zweite Rolle in der Führungsnut gleiten kann, sodass der Verbindungsarm die Drucklagerkomponente (22) antreibt, um sie in die Drückrichtung zu bewegen.

11. Elektronische Vorrichtung nach Anspruch 10, wobei sich die zweite Führungsnut in dem Montagehohlraum (111) befindet; oder
die zweite Führungsnut in einen Hohlraum, der mit dem Montagehohlraum (111) in Kommunikation steht, ausgebildet ist.

12. Elektronische Vorrichtung nach einem der Ansprüche 1 bis 11, wobei das Armband (11) ein Metallarmband oder ein Fluorkautschukarmband ist.

## Revendications

1. Dispositif électronique portable (100), comprenant :
un élément de pressage (20) ;
un capteur de pression (30) ;
dans lequel l'élément de pressage (20) comprend un élément d'entraînement électromagnétique (21) et un élément de support de pression (22) ; dans lequel l'élément de support de pression (22) peut se déplacer sous l'effet d'une force électromagnétique de l'élément d'entraînement électromagnétique (21) ;
le capteur de pression (30) est disposé sur l'élément de support de pression (22), et le capteur de pression (30) est situé sur une surface de l'élément de support de pression (22) et qui fait face à une partie de corps de mesure d'un utilisateur ;
l'élément de support de pression (22) est configuré pour être entraîné par l'élément d'entraînement électromagnétique (21) afin de presser le capteur de pression (30) sur la partie de corps de mesure, et
le capteur de pression (30) est configuré pour détecter un signal d'onde de pouls de la partie de corps de mesure,
dans lequel le dispositif électronique comprend un bracelet (11) et un corps électronique (12), le bracelet (11) est relié au corps électronique (12) et l'élément de pression (20) est disposé dans le bracelet (11),
dans lequel une cavité de montage est disposée sur une surface qui se trouve dans le bracelet (11) et qui fait face à la partie de corps de mesure, et au moins une partie de l'élément de pressage (20) est disposée dans la cavité de montage (111),
dans lequel l'élément de support de pression (22) est relié de manière coulissante à une paroi intérieure de la cavité de montage (111).

2. Dispositif électronique selon la revendication 1, dans lequel, lorsque le capteur de pression (30) est pressé sur la partie de corps de mesure par l'élément de support de pression (22), une direction de mouvement de l'élément de support de pression (22) va de l'élément d'entraînement électromagnétique (21) vers la partie de corps de mesure.

3. Dispositif électronique selon la revendication 1 ou 2, dans lequel l'un parmi l'élément d'entraînement électromagnétique (21) et l'élément de support de pression (22) comprend un électro-aimant, et l'autre comprend un aimant ; et des pôles magnétiques de l'électro-aimant et des pôles magnétiques de l'aimant sont opposés l'un à l'autre ; et l'électro-aimant a une polarité réglable, de sorte que lorsque des pôles de l'électro-aimant et des pôles de l'aimant se repoussent, le capteur de pression (30) est pressé sur la partie de corps de mesure, ou lorsque des pôles de l'électro-aimant et des pôles de l'aimant s'attirent, le capteur de pression (30) quitte la partie de corps de mesure.

4. Dispositif électronique selon la revendication 3, dans lequel l'élément de support de pression (22) est relié de manière fixe au capteur de pression (30) ou est relié de manière amovible au capteur de pression (30).

5. Dispositif électronique selon la revendication 3 ou 4, dans lequel l'électro-aimant comprend un noyau magnétique et une bobine enroulée autour d'un côté périphérique extérieur du noyau magnétique.

6. Dispositif électronique selon l'une quelconque des revendications 1 à 5, comprenant en outre au moins un élément coulissant, dans lequel l'élément coulissant est relié entre l'élément de support de pression (22) et la paroi intérieure de la cavité de montage (111), de sorte que l'élément de support de pression (22) est relié de manière coulissante à la paroi intérieure de la cavité de montage (111).

7. Dispositif électronique selon la revendication 6, dans lequel chaque élément coulissant comprend une première poulie disposée sur une paroi latérale de l'élément de support de pression (22) et une glissière disposée sur la paroi intérieure de la cavité de montage (111), la poulie et la glissière sont disposées à l'opposé l'une de l'autre, et la première poulie peut coulisser le long de la glissière, de sorte que l'élément de support de pression (22) est relié de manière coulissante à la paroi intérieure de la cavité de montage (111).

8. Dispositif électronique selon la revendication 7, dans lequel une pièce de limitation est disposée au niveau d'une ouverture de la cavité de montage (111), et la pièce de limitation est disposée de manière correspondante à l'extérieur de la première poulie dans une direction de pression.

9. Dispositif électronique selon la revendication 7 ou 8 et selon la revendication 3, dans lequel un élément de guidage est en outre disposé entre l'élément de support de pression (22) et l'élément d'entraînement électromagnétique (21), une première rainure de guidage correspondant à l'élément de guidage est en outre disposée dans l'élément d'entraînement électromagnétique (21), une première extrémité de l'élément de guidage est reliée à l'aimant, et une seconde extrémité de l'élément de guidage passe à travers la première rainure de guidage et peut se déplacer dans une direction d'extension de la première rainure de guidage.

10. Appareil électronique selon la revendication 8, dans lequel l'élément coulissant comprend une seconde poulie, un bras de liaison et une seconde rainure de guidage, la seconde rainure de guidage et la cavité de montage (111) sont fixées l'une par rapport à l'autre, et une direction d'extension de la seconde rainure de guidage est décalée par rapport à une direction de pression de l'élément d'entraînement électromagnétique (21) ; une première extrémité du bras de liaison est articulée avec l'élément de support de pression (22), la seconde poulie est disposée au niveau d'une seconde extrémité du bras de liaison, et la seconde poulie peut coulisser dans la rainure de guidage, de sorte que le bras de liaison entraîne l'élément de support de pression (22) à se déplacer dans la direction de pressage.

11. Dispositif électronique selon la revendication 10, dans lequel la seconde rainure de guidage est située dans la cavité de montage (111) ; ou
la seconde rainure de guidage est formée dans une cavité en communication avec la cavité de montage (111).

12. Dispositif électronique selon l'une quelconque des revendications 1 à 11, dans lequel le bracelet (11) est un bracelet en métal ou en caoutchouc fluoré.
